# EUROPEAN PATENT APPLICATION

(11) **EP 3 556 372 A1**
(43) Date of publication of application: **23.10.2019**
(21) Application number: 19159190.8
(22) Date of filing: 14.09.2012
(51) Int. Cl.: A61K 33/18, A61K 31/196, A61K 45/06, A61P 27/02, A61P 31/00

(54) **STABLE POVIDONE-IODINE COMPOSITIONS**

(30) Priority: 16.09.2011 US 201161535667 P
(62) Divisional of application: 12832369.8
(71) Applicant: Foresight Biotherapeutics, Inc., New York, NY 10019 (US)
(72) Inventor: CAPRIOTTI, Joseph, 00820 Christiansted (VI); LIANG, Bo, Plainsboro, New jersey 08536 (US); SAMSON, C. Michael, New York, NY New York 10016 (US); STEIN, Jason, New York, NY New York 10162 (US); WEISER, Michael, New York, NY New York 10012 (US)
(74) Representative: Carpmaels & Ransford LLP

(57) **Abstract**

Disclosed herein are PVP-I-containing compositions, as well as methods of making such compositions, which provide reliable stability for PVP-I preparations, including preparations comprising PVP-I and one or more additional components.

## Description

### BACKGROUND

Povidone-iodine ("PVP-I") has various uses, including treatment of burns and of different skin lesions (e.g., decubitus and leg ulcers). In some preparations, it is available for the therapy of inflammations in the ear, mouth and pharynx, and for vaginitis. PVP-I is used in the treatment of skin disinfection in the prevention of nosocomial infections, especially, prior to invasive procedures such as the insertion of peripheral catheters, treatment of exit site infection.

PVP-I is effective against variety of microorganisms, including bacteria, viruses, and fungi. The use of PVP-I can circumvent problems found with more traditional antibiotics, such as antibiotic resistance and problems with compound versatility. For example, otitis media (middle ear infection) occurs in the area between the ear drum and the inner ear, including the Eustachian tube. Ear infection (particularly in children) is one of the many diseases that have become hard to treat with traditional antibiotic drugs because of antibiotic resistant bacteria and antibiotic-resistant microorganisms. Most cases of otitis media, for example, are caused by one of several major pathogens, Streptococcus pneumonia, Haemophilus influenza, Moraxella catarrhalia, Staphylococcus aureus, Staphylococcus epidermidis, or Pseudomonas aeruginosa. PVP-I is effective against these organisms, and in one example, would be useful for treatment of otitis media in cases in which the tympanic membrane is breeched or damaged, to allow penetration of the PVP-I solution.

However, PVP-I solutions sometimes lack predictable stability. Furthermore, the combination of PVP-I with other components is well-documented to render the PVP-I unpredictably unstable. For example, PVP-I is useful for treatment of ophthalmic conditions. In U.S. Patent 7,767,217, it is shown that under certain specific conditions, dexamethasone can be combined with PVP-I to form an effective antimicrobial-steroid pharmaceutical composition. However, it is also shown that most preparations which combine PVP-I (or iodine) with a steroid suffer from instability due, in part, to reactivity of the iodine with the steroid. In fact, U.S. Patent 3,886,268 demonstrates the well-known instability of steroid-iodine combinations.

### SUMMARY

In an embodiment, a composition is provided that is suitable for topical administration, comprising povidone-iodine (PVP-I) at a starting concentration between about 0.4% and about 12.5% by weight, wherein after a period of one month after preparing the composition, the PVP-I concentration is at least 98% of the PVP-I starting concentration, and after a period of six months after preparing the composition, the PVP-I concentration is at least 96% of the PVP-I starting concentration.

In an embodiment, a composition is provided that is suitable for topical administration, comprising a mixture of PVP-I at a starting concentration between about 0.4% and about 12.5% by weight and at least one non-steroidal anti-inflammatory (NSAID) selected from the group consisting of amfenac, bromfenac, ketotifen fumarate, diclofenac, diclofenac sodium, flurbiprofen sodium, ketorlac, ketorlac tromethamine, suprofen, celecoxib, naproxen, rofecoxib, and combinations and salts thereof, wherein after a period of one month after mixing the NSAID and PVP-I to form the composition, the PVP-I concentration is at least 98% of the PVP-I starting concentration, and after a period of six months after mixing the NSAID and PVP-I to form the composition, the PVP-I concentration is at least 96% of the PVP-I starting concentration.

In an embodiment, a composition is provided that is suitable for topical administration, comprising a mixture of PVP-I at a starting concentration between about 0.4% and about 12.5% by weight and at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof, wherein after a period of one month after mixing the steroid and PVP-I to form the composition, the PVP-I concentration is at least 98% of the PVP-I starting concentration, and after a period of six months after mixing the steroid and PVP-I to form the composition, the PVP-I concentration is at least 96% of the PVP-I starting concentration.

In an embodiment, a composition is provided that is suitable for topical administration, comprising a mixture of PVP-I at a starting concentration between about 0.4% and about 12.5% by weight, and at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof, wherein after a period of one month after mixing the steroid and PVP-I to form the composition, the PVP-I concentration is at least 98% of the PVP-I starting concentration, and after a period of six months after mixing the steroid and PVP-I to form the composition, the PVP-I concentration is at least 96% of the PVP-I starting concentration, further wherein, after a period of one month after mixing the steroid and PVP-I to form the composition, the steroid concentration is at least 90% of the steroid starting concentration.

In an embodiment, a method is provided for treating a mammal having an otic infection, the method comprising contacting the ear of the mammal with a composition disclosed herein.

In an embodiment, a composition is provided that is suitable for topical administration, comprising PVP-I at a starting concentration between about 0.001 % and about 0.6% by weight, wherein after a period of one month after preparing the composition, the PVP-I concentration is at least 93% of the PVP-I starting concentration, and after a period of six months after preparing the composition, the PVP-I concentration is at least 93% of the PVP-I starting concentration.

In an embodiment, a composition is provided that is suitable for topical administration, comprising a mixture of PVP-I at a starting concentration between about 0.001% and about 0.6% by weight, and at least one NSAID selected from the group consisting of amfenac, bromfenac, ketotifen fumarate, diclofenac, diclofenac sodium, flurbiprofen sodium, ketorlac, ketorlac tromethamine, suprofen, celecoxib, naproxen, rofecoxib, and combinations and salts thereof, wherein after a period of one month after mixing the NSAID and PVP-I to form the composition, the PVP-I concentration is at least 93% of the PVP-I starting concentration, and after a period of six months after mixing the NSAID and PVP-I to form the composition, the PVP-I concentration is at least 93% of the PVP-I starting concentration.

In an embodiment, a composition is provided that is suitable for topical administration, comprising a mixture of PVP-I at a starting concentration between about 0.001 % and about 0.6% by weight, and at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof, wherein after a period of one month after mixing the steroid and PVP-I to form the composition, the PVP-I concentration is at least 93% of the PVP-I starting concentration, and after a period of six months after mixing the steroid and PVP-I to form the composition, the PVP-I concentration is at least 93% of the PVP-I starting concentration.

In an embodiment, a composition is provided that is suitable for topical administration, comprising a mixture of PVP-I at a starting concentration between about 0.001 % and about 0.6% by weight, and at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof, wherein after a period of one month after mixing the steroid and PVP-I to form the composition, the PVP-I concentration is at least 93% of the PVP-I starting concentration, and after a period of six months after mixing the steroid and PVP-I to form the composition, the PVP-I concentration is at least 93% of the PVP-I starting concentration, further wherein, after a period of one month after mixing the steroid and PVP-I to form the composition, the steroid concentration is at least 90% of the steroid starting concentration.

In an embodiment, a method for treating an eye disorder or a microorganism infection of at least one tissue of the eye comprising the step of administering one or more doses of a composition disclosed herein to the eye.

In an embodiment, an ophthalmic composition is provided that is suitable for topical administration to an eye, effective for treatment and/or prophylaxis of a microorganism infection or a disorder of at least one tissue of the eye, comprising a mixture of PVP-I at a starting concentration between about 0.4% and about 1.0% by weight, and at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof, wherein after a period of one month after mixing the steroid and PVP-I to form the composition, the PVP-I concentration is at least 98% of the PVP-I starting concentration, and after a period of six months after mixing the steroid and PVP-I to form the composition, the PVP-I concentration is at least 96% of the PVP-I starting concentration.

In an embodiment, an ophthalmic composition is provided that is suitable for topical administration to an eye, effective for treatment and/or prophylaxis of a microorganism infection or a disorder of at least one tissue of the eye, comprising a mixture of PVP-I at a starting concentration between about 0.1% and about 0.6% by weight, and at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof, wherein after a period of one month after mixing the steroid and PVP-I to form the composition, the PVP-I concentration is at least 93% of the PVP-I starting concentration, and after a period of six months after mixing the steroid and PVP-I to form the composition, the PVP-I concentration is at least 93% of the PVP-I starting concentration.

In an embodiment, a composition is provided that is suitable for topical administration, comprising PVP-I at a starting concentration between about 0.4% and about 12.5% by weight, wherein after a period of one month after preparation of the composition, the PVP-I concentration is within about 2% to about 3% of the PVP-I starting concentration, and after a period of six months after preparation of the composition, the PVP-I concentration is within about 2% to about 3% of the PVP-I concentration at one month after preparation of the composition.

In an embodiment, a composition is provided that is suitable for topical administration, comprising PVP-I at a starting concentration between about 0.001% and about 0.6% by weight, wherein after a period of one month after preparation of the composition, the PVP-I concentration is about 5% to about 10% below the PVP-I starting concentration, and after a period of six months after preparation of the composition, the PVP-I concentration is within about 1% of the PVP-I concentration at one month after preparation of the composition.

### DETAILED DESCRIPTION

It is known that iodine, including preparations of PVP-I, can react chemically with various substances, making iodine in solution unstable. As shown in U.S. Patent No. 5,126,127, incorporated herein by reference in its entirety, PVP-I solutions have been packaged for medicinal use, e.g. in soft plastic bottles or containers, which can be used for various medicinal purposes. However, one problem that has been encountered with such packaged iodophor solutions is that elemental iodine (equilibrium iodine) has leached through the packaging itself. In the past, this resulted both in a decrease in stability and medicinal capacity of the iodophor solution contained within the packaging, and made it difficult to handle such packaging since the elemental iodine which leached through caused staining, and in some cases, leakage. The problems associated with packaging such PVP-I solutions in soft plastic bottles or containers have been overcome through the addition of other stabilizers or iodine donating species such as iodate salts, as disclosed in U.S. Pat. No. 4,113,857, and the use of iodide salts, as disclosed in U.S. Pat. No. 4,996,048. However, the addition of unwanted components is undesirable, and sometimes creates undesirable side effects and increases product cost.

Although PVP-I solutions are known to exert microbicidal activity, stabilizing PVP-I solutions for various uses (e.g., ophthalmic use) can be problematic. Furthermore, in view of the stability problems associated with dilute PVP-I solutions, it is difficult to provide an acceptable formulation for dilute PVP-I solutions, such as for ophthalmic use. For example, the introduction of donating species such as iodate into a PVP-I solution is not considered to be desirable when the solution is to be used as an ophthalmic preparation because iodate and iodide are known to be irritating and toxic to the pigment epithelium of the retina. Thus, a PVP-I solution stabilized via the addition of, for example, potassium iodide and/or potassium iodate would not be useful as an ophthalmic preparation.

Furthermore, compositions comprising PVP-I and a steroid may suffer from instability due to the reactivity between iodine and the steroid. The affinity of free iodine for reaction with --OH, --SH and --NH functional groups is well described in the literature and forms the basis for the anti-microbial activity of iodine-containing solutions (Rackur H. J. Hosp. Infect., 1985; 6: 13-23, and references therein). Dexamethasone, (9-Fluoro-11.beta., 17, 21-trihydroxy-16.alpha.-methylpregna-1, 4-diene-3,20-dione) for example, contains three such moieties (--OH) at the 11, 17 and 21 positions. The skilled artisan would conclude that these hydroxyl groups would be prone to covalent substitution reactions by the free iodine generated in the solution equilibrium reaction described above for PVP-I.₂.

The compositions and methods disclosed herein provide reliable stability for PVP-I preparations, including preparations comprising PVP-I and one or more additional components. The characteristics of the stability of a PVP-I composition encompassed herein are referred to herein as a "stability profile".

In an aspect, the PVP-I compositions disclosed herein demonstrate stability through a multi-phasic degradation mechanism. In another aspect, the PVP-I compositions disclosed herein demonstrate stability through a multi-phasic degradation mechanism, with reference to the time at which the composition is prepared. In an embodiment, a PVP-I composition demonstrates stability by way of a biphasic degradation pattern. In an embodiment, PVP-I degrades at a first rate in the first phase, followed by a second phase in which PVP-I degrades more slowly in comparison to the rate of degradation during the first phase.

"Stability", as the term is used herein, refers to the degradation of PVP-I. In particular, degradation of PVP-I includes, among other things, the loss of iodine from PVP-I.

"Compatibility", as the term is used herein, refers to the ability of a substance to coexist in a composition with PVP-I, without being oxidized by PVP-I.

In an aspect, a PVP-I composition demonstrates stability by way of a unique biphasic degradation pattern depending upon the starting concentration of the PVP-I composition. In an embodiment, the rate of degradation of PVP-I in the first phase, the rate of PVP-I degradation in the second phase, and the relative rate of degradation of PVP-I in the first and second phases all may differ, either independently or dependent upon one another, based upon the starting concentration. In an embodiment, the stability profile of a PVP-I composition may be affected by the addition to or the removal of any additional components from the composition. In another embodiment, the stability profile of a PVP-I composition may be affected by the concentration of any additional components in the composition. In an embodiment, the stability profile of a PVP-I composition may be affected by one or more of stirring, agitation, application of heat, cooling of the composition, or by the adjustment of any physical or chemical parameter of the composition.

### Compositions

In an embodiment, disclosed herein is composition comprising PVP-I at a starting concentration between about 0.4% and about 12.5% by weight, wherein after a period of one month after preparing the composition, the PVP-I concentration is at least 98% of the PVP-I starting concentration, and after a period of six months after preparing the composition, the PVP-I concentration is at least 96% of the PVP-I starting concentration. In another embodiment, disclosed herein is a composition comprising PVP-I at a starting concentration between about 0.001 % and about 0.6% by weight, wherein after a period of one month after preparing the composition, the PVP-I concentration is at least 93% of the PVP-I starting concentration, and after a period of six months after preparing the composition, the PVP-I concentration is at least 93% of the PVP-I starting concentration.

In an embodiment, the composition further comprises an NSAID.

In another embodiment, the composition further comprises a steroid. In an embodiment, after a period of one month after mixing the steroid and PVP-I to form the composition, the steroid concentration is at least 90% of the steroid starting concentration.

The compositions disclosed herein are useful for topical administration, including, but not limited to, application to the eye, the skin, the ear, nasal passages, sinuses, and the vagina.

In an embodiment, a composition comprises PVP-I at a concentration in the range of about 0.001% to about 0.75%. In another embodiment, a composition comprises PVP-I at a concentration in the range between 0.005% and 0.7%. In another embodiment, a composition comprises PVP-I at a concentration in the range between 0.01% and 0.65%. In another embodiment, a composition comprises PVP-I at a concentration in the range between 0.05% and 0.6%. In another embodiment, a composition comprises PVP-I at a concentration in the range between 0.1% and 0.5%. In another embodiment, a composition comprises PVP-I at a concentration in the range between 0.1% and 0.4%, and in yet another embodiment, between 0.1% and 0.3%. In an embodiment, a composition comprises PVP-I at a concentration in the range of about 0.1% to about 0.25%, about 0.1% to about 0.2%, and about 0.1% to about 0.15%.

In an embodiment, a composition comprises PVP-I at a concentration in the range of about 0.3% to about 12.5%. In another embodiment, a composition comprises PVP-I at a concentration in the range between 0.4% and 12.5%. In another embodiment, a composition comprises PVP-I at a concentration in the range between 0.5% and 12.5%. In another embodiment, a composition comprises PVP-I at a concentration in the range between 0.6% and 12.5%. In another embodiment, a composition comprises PVP-I at a concentration in the range between 0.7% and 12.5%. In another embodiment, a composition comprises PVP-I at a concentration in the range between 0.8% and 12.5%, and in yet another embodiment, between 0.9% and 12.5%. In an embodiment, a composition comprises PVP-I at a concentration in the range of about 1.0% to about 12.5%, about 2.0% to about 12.5%, about 3.0% to about 12.5%, about 4.0% to about 12.5%, about 5.0% to about 12.5%, about 7.5% to about 12.5%, and about 10.0% to about 12.5%.

In an embodiment, a composition comprises PVP-I at a concentration in the range of about 0.001% to about 12.5%. In another embodiment, a composition comprises PVP-I at a concentration in the range between 0.01% and 10.0%. In another embodiment, a composition comprises PVP-I at a concentration in the range between 0.05% and 7.5%. In another embodiment, a composition comprises PVP-I at a concentration in the range between 0.1% and 5.0%. In another embodiment, a composition comprises PVP-I at a concentration in the range between 0.1% and 2.5%. In another embodiment, a composition comprises PVP-I at a concentration in the range between 0.2% and 1.5%, and in yet another embodiment, between 0.3% and 1.0%. In an embodiment, a composition comprises PVP-I at a concentration in the range of about 0.2% to about 2.0%, about 0.3% to about 1.5%, about 0.36% to about 1.0%, and about 0.4% to about 0.75%.

In an embodiment, a composition comprises PVP-I at a concentration of about 0.001%, about 0.005%, about 0.01%, about 0.05%, about 0.1%, about 0.2%, about 0.3%, about 0.4%, about 0.5%, about 0.6%, about 0.7%, about 0.8%, about 0.9%, about 1.0%, about 2.5%, about 5%, about 7.5%, about 10%, or about 12.5%. In an embodiment, a composition comprises povidone-iodine PVP-I at a concentration of 0.001%, 0.005%, 0.01%, 0.05%, 0.1%, 0.2%, 0.3%, 0.4%, 0.5%, 0.6%, 0.7%, 0.8%, 0.9%, 1.0%, 2.5%, 5%, 7.5%, 10.0%, or 12.5%. In another embodiment, a composition comprises PVP-I at a concentration of about 1%, about 2%, about 3%, about 4%, about 5%, about 6%, about 7%, about 8%, about 9%, about 10%. In another embodiment, a composition comprises PVP-I at a concentration of about 2% or less, about 3% or less, about 4% or less, about 5% or less, about 6% or less, about 7% or less, about 8% or less, about 9% or less or about 10% or less. In another embodiment, a composition comprises PVP-I at a concentration of about 0.1% or more, about 0.2% or more, about 0.3% or more, about 0.4% or more, about 0.5% or more, about 0.6% or more, about 0.7% or more, about 0.8% or more, about 0.9% or more, 1% or more, about 2% or more, about 3% or more, about 4% or more, about 5% or more, about 6% or more, about 7% or more, about 8% or more, about 9% or more or about 10% or more. In another embodiment, a composition comprises PVP-I at a concentration of 0.001%, 0.005%, 0.01%, 0.05%, 0.1%. 0.2%, 0.3%, 0.4%, 0.5%, 0.6%, 0.7%, 0.8%, 0.9%, 1.0%, 2.0%, 3.0%, 4.0%, 5.0%, 6.0%, 7.0%, 8.0%, 9.0% or 10.0%.

Compositions disclosed herein may further comprise one or more additional components.

In an embodiment, compositions disclosed herein comprise PVP-I and a steroid. In another embodiment, a composition disclosed herein is a pharmaceutical composition. In another embodiment, a composition disclosed herein is an ophthalmic composition.

In an embodiment, compositions disclosed herein may further comprise one or more steroids. Steroids include, but are not limited to, dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and any combinations thereof. The steroid may be used any form, and in various modified forms such as acetate forms, and sodium phosphate forms, sodium salts, and the like. In an embodiment, a pharmaceutically acceptable salt of the steroid is used.

In an embodiment, compositions disclosed herein may further comprise one or more non-steroidal anti-inflammatory compounds (NSAIDS). NSAIDS include, but are not limited to, amfenac, bromfenac, ketotifen fumarate, diclofenac, diclofenac sodium, flurbiprofen sodium, ketorlac, ketorlac tromethamine, suprofen, celecoxib, naproxen, rofecoxib, or a derivative or combination thereof. Pharmaceutically-acceptable salts of NSAIDS are also contemplated herein.

In an embodiment, a steroid and/or NSAID is present in the composition at a level of about 0.001% to about 10%. In an embodiment, a steroid and/or NSAID is present in the composition or preparation at a level of 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, or 2.0%. In an embodiment, a steroid and/or NSAID is present in the composition or preparation at a level of about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, or about 2.0%. In an embodiment, a steroid and/or NSAID is present in the composition or preparation at a level of about 0.001% or less, about 0.002% or less, about 0.003% or less, about 0.004% or less, about 0.005% or less, about 0.006% or less, about 0.007% or less, about 0.008% or less, about 0.009% or less, about 0.01% or less, about 0.02% or less, about 0.03% or less, about 0.04% or less, about 0.05% or less, about 0.06% or less, about 0.07% or less, about 0.08% or less, about 0.09% or less, about 0.1% or less, about 0.2% or less, about 0.3% or less, about 0.4% or less, about 0.5% or less, about 0.6% or less, about 0.7% or less, about 0.8% or less, about 0.9% or less, about 1.0% or less, about 1.1% or less, about 1.2% or less, about 1.3% or less, about 1.4% or less, about 1.5% or less, about 1.6% or less, about 1.7% or less, about 1.8% or less, about 1.9% or less, or about 2.0% or less. In an embodiment, a steroid and/or NSAID is present in the composition or preparation at a level of about 0.001% or more, about 0.002% or more, about 0.003% or more, about 0.004% or more, about 0.005% or more, about 0.006% or more, about 0.007% or more, about 0.008% or more, about 0.009% or more, about 0.01% or more, about 0.02% or more, about 0.03% or more, about 0.04% or more, about 0.05% or more, about 0.06% or more, about 0.07% or more, about 0.08% or more, about 0.09% or more, about 0.1% or more, about 0.2% or more, about 0.3% or more, about 0.4% or more, about 0.5% or more, about 0.6% or more, about 0.7% or more, about 0.8% or more, about 0.9% or more, about 1.0% or more, about 1.1% or more, about 1.2% or more, about 1.3% or more, about 1.4% or more, about 1.5% or more, about 1.6% or more, about 1.7% or more, about 1.8% or more, about 1.9% or more, or about 2.0% or more.

The compositions disclosed herein can be administered as solutions, suspensions, emulsions (dispersions), gels, creams, or ointments in a suitable ophthalmic vehicle. In any of the compositions of this disclosure for topical administration, such as topical administration to the eye, the mixtures are preferably formulated as aqueous solutions at a pH of 3.5 to 6.5. Preferentially the pH is adjusted to between 4 and 5. This pH range may be achieved by the addition of acids/bases to the solution.

In an embodiment, an ophthalmic composition may comprise an optional co-solvent. In another embodiment, the solubility of the components of the present compositions may be enhanced by a surfactant or other appropriate co-solvent in the composition. Such co-solvents or surfactants include polysorbate -20, -60, and -80, a polyoxyethylene/polyoxypropylene surfactant (e.g. Pluronic F-68, F-84 and P-103), cyclodextrin, tyloxapol, PEG 35 Castor oil (Cremophor EL), polyoxyl 40 Stearate (Myrj 52), other agents known to those skilled in the art, or a combination thereof. Typically, such co-solvents are present at a level of from about 0.01% to about 2% by weight. In an embodiment, a co-solvent is present at a level of about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, or about 2.0%.

In an embodiment, a composition may comprise an optional agent that can increase viscosity. As will be understood by the skilled artisan when armed with the present disclosure, it may be desirable to increase viscosity above that of a simple aqueous solution in order to increase ocular absorption of the active compound, to decrease variability in dispensing the formulation, to decrease physical separation of components of a suspension or emulsion of the formulation and/or to otherwise improve the ophthalmic formulation. Such viscosity-enhancing agents include, but are not limited to, polyvinyl alcohol, polyvinyl pyrrolidone, methyl cellulose, hydroxypropylmethyl cellulose, hydroxyethyl cellulose, carboxymethyl cellulose, hydroxypropyl cellulose, other agents known to those skilled in the art, or any combination thereof. Such agents are typically employed at a level of from about 0.01% to about 2% by weight. In an embodiment, such optional agents are present at about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, or about 2.0%.

In another aspect, bioadhesive agents may comprise the compositions, in order to increase the retention time of the drug gradient over a biological substrate. The bioadhesive agents include, but are not limited to, polyvinylpyrrolidone (PVP), xanthan gum, locust bean gum, acacia gum, hydroxypropyl methylcellulose (HPMC), sodium alginate, pectin, gelatin, carbomer, polyvinylalcohol, gellan gum, tragacanth, acacia, and sodium carboxymethyl cellulose, as well as other agents known to those skilled in the art, or any combination thereof. In yet another embodiment, compositions of the invention may comprise viscoelastic agents such as methyl cellulose, carboxymethyl cellulose, hydroxyethyl cellulose, polyvinyl alcohol, dextran, chondroitin sulfate and salts thereof, and hyaluronic acid and salts thereof.

Compositions disclosed herein may be buffered or non-buffered. The skilled artisan will understand when a PVP-I composition may require buffering, or when a method of use will benefit from a buffered PVP-I composition.

In an embodiment, a composition disclosed herein may consist essentially of PVP-I. In an embodiment, a composition disclosed herein may consist essentially of PVP-I and one or more steroids. In an embodiment, a composition disclosed herein may consist essentially of PVP-I and one or more NSAIDS. In an embodiment, a composition disclosed herein may consist essentially of PVP-I and one or more steroids and one or more NSAIDS. In an aspect, a composition consisting essentially of PVP-I, PVP-I plus one or more steroids, PVP-I plus one or more NSAIDS, or PVP-I plus one or more steroids and one or more NSAIDS does not contain any other components that materially affect the basic and novel characteristics of the composition. In another aspect, a composition consisting essentially of PVP-I, PVP-I plus one or more steroids, PVP-I plus one or more NSAIDS, or PVP-I plus one or more steroids and one or more NSAIDS does not contain any other components that materially affect the basic and novel characteristics of the method of use of the composition. In yet another aspect, a composition consisting essentially of PVP-I, PVP-I plus one or more steroids, PVP-I plus one or more NSAIDS, or PVP-I plus one or more steroids and one or more NSAIDS does not contain any other components that materially affect the basic and novel characteristics of the composition, but may affect the method of use of the composition such that the composition may have the same basic effect on a subject, but that the composition may provide one or more of fewer side effects, less severe side effects, increased efficacy, decreased toxicity, more rapid treatment of the adverse health condition, more complete treatment of the adverse health condition, and the ability to use or administer the composition in conjunction with one or more other compositions.

It will be understood that the balance of a composition, after addition of the one or more components specified herein, may be water, or other suitable solvent or carrier. Other components necessary to prepare a suitable pharmaceutical composition can also be included in addition to the one or more components specified herein.

### Methods

In an embodiment, compositions disclosed herein are useful for preparation of and use as pharmaceutical compositions. In another embodiment, compositions disclosed herein are useful for preparation of and use as compositions other than pharmaceutical compositions.

Disclosed herein is a method for treating an eye disorder, or a microorganism infection of at least one tissue of the eye, comprising the step of administering one or more doses of a composition disclosed herein to the eye. In an embodiment, compositions disclosed herein are useful for preparation of and use as ophthalmic compositions. In an aspect, a composition of the invention is useful in the treatment of infections of the conjunctiva and cornea. In another aspect, the broad spectrum antimicrobial activity of povidone-iodine enables a composition of the invention to be used to treat ocular conjunctival or corneal infection caused by mycobacteria, viruses, fungi, and amoeba. Additionally the composition is useful in the infectious prophylaxis of patients recovering from ophthalmic surgery.

In an embodiment, an ophthalmic composition is provided that is suitable for topical administration to an eye, effective for treatment and/or prophylaxis of a microorganism infection or a disorder of at least one tissue of the eye. Prophylaxis may be, for example, prophylaxis from infection following surgery, prophylaxis from infection after birth for the newborn, or prophylaxis from accidental contact with contaminating material. Accidental contact with contaminating material may occur, for example, during surgery or through close contact with a contaminated family member or co-worker.

In an embodiment, an ophthalmic composition may further comprise one or more of (1) a penetration enhancer which enhances the penetration of povidone-iodine into the tissues of the eye (this may be a topical anesthetic) (2) a co-solvent or a nonionic surface agent - surfactant, which, for example, may be about 0.01% to 2% by weight; (3) a viscosity increasing agent, which, for example, may be about 0.01% to 2% by weight; and (4) a suitable ophthalmic vehicle.

The ophthalmic composition may be in the form of a solution, a suspension, an emulsion, a preparation, an ointment, a cream, a gel, or a controlled-release/sustain-release vehicle. By way of a non-limiting example, the composition may be in the form of a contact lens solution, eyewash, eyedrop, and the like.

In an aspect, the ophthalmic composition may be used for treatment and/or prophylaxis of a microorganism infection. The microorganism may be a bacterium, a virus, a fungus, or an amoeba, a parasite, or a combination thereof. In an embodiment, the bacteria may be a mycobacterium.

In an aspect, an ophthalmic composition may be used to treat a disorder such as, but not limited to, conjunctivitis, corneal abrasion, ulcerative infectious keratitis, epithelial keratitis, stromal keratitis, herpesvirus-related keratitis, ocular surface irregularity, tear deficiency, dry syndrome, meibomian gland dysfunction, blepharitis and uveitis. In another aspect, an ophthalmic composition may be used for prophylaxis of disorders such as conjunctivitis, corneal abrasion, ulcerative infectious keratitis, epithelial keratitis, stromal keratitis, herpesvirus-related keratitis, ocular surface irregularity, tear deficiency, dry syndrome, meibomian gland dysfunction, blepharitis and uveitis.

In another embodiment, the invention is directed to a method for treating and/or prophylaxis of an eye disorder or a microorganism infection of at least one tissue of the eye comprising the step of administering one of more doses of an ophthalmic composition, discussed above, to the eye. The eye disorder may be, for example, a microorganism infection of at least one tissue of the eye, conjunctivitis, corneal abrasion, ulcerative infectious keratitis, epithelial keratitis, stromal keratitis, herpes virus-related keratitis, ocular surface irregularity, tear deficiency, dry syndrome, meibomian gland dysfunction, and blepharitis. The microorganism may be bacteria (e.g., mycobacteria), virus, fungi, or amoebae.

In an embodiment, the dose volume administered to a subject may be between about 10 microliters and about 200 microliters, in another embodiment, between about 20 microliters and 100 microliters, and in another embodiment, between about 50 microliters and about 80 microliters, or about one drop per eye. Two or more drops may be added to an eye. Treatment of an eye may be effected by adding a single drop of composition disclosed herein, or by adding two or more drops, as required to achieve the desired result.

In an embodiment, administration frequency may be between 1 and 24 times a day. In an embodiment, administration frequency may be between 1 and 48 times a day. In another embodiment, administration frequency may be between 2 and 24 times a day. In another embodiment, administration frequency may be between 2 and 4 times a day. In another embodiment, administration frequency may be twice a day. In another embodiment, administration frequency may be once a day. In another embodiment, administration frequency may be less frequent than once a day. In another embodiment, administration frequency may be on demand, as therapeutic treatment is required or desired. In another embodiment, administration frequency may be 1, 2, 3, 4, 5, 6, 7, 8, 9, 10, 11, 12, 13, 14, 15, 16, 17, 18, 19, 20, 21, 22, 23, 24, 48, or 96 times a day.

In an embodiment, a composition disclosed herein is used for prophylaxis and/or treatment of a non-ophthalmic tissue by contacting the tissue with the composition.

Also disclosed herein is a method of treating a mammal having an otic infection, comprising contacting the ear of the mammal with a composition as disclosed herein. In an aspect, topical ear medications are not typically able to penetrate the tympanic membrane thus limiting their usefulness and effectiveness. However, in cases of recurrent otitis media it is common for physicians to place ventilation tubes through the tympanic membrane in an effort to reduce the pressure built up in the middle ear and to allow the middle ear space to "dry out". Quite frequently, this middle ear space becomes infected again and because of the placement of the ventilation tube topical preparations have access to the middle ear infection. Another non-limiting example in which topically applied medications have access to the middle ear is in chronic suppurative otitis media, in which a long-standing ear infection has caused the perforation of the tympanic membrane. In clinical conditions such as these, it is possible to treat the underlying middle ear infection with topical medications. By way of another non-limiting example, it is possible to treat other clinical conditions with topical medications. In an embodiment, in cases of otitis externa in which the tympanic membrane is intact and the infection is solely located external to the tympanic membrane, topical medications have demonstrated clinical utility and are used widely.

In an embodiment, disclosed herein is a method of using a topical pharmaceutical composition for treating and relieving the symptoms of ear, including, but not limited to, otitis interna, otitis media and otitis externa (both acute and chronic). In an embodiment, the compositions comprise PVP-I in an amount effective to reduce the growth of infection causing microbes and a pharmaceutically acceptable carrier therefor. In an embodiment, PVP-I is present in an otic composition in the range of about 0.1 %-10%, about 0.5%-5%, or about 1% to about 3%. In an embodiment, PVP-I is present in an otic composition at about 2%. Other suitable PVP-I concentrations are set forth elsewhere herein. In an embodiment, the otic compositions may additionally comprise a steroid, such as, but not limited to, dexamethasone.

Methods of treating a mammal for an otic infection use the compounds disclosed herein. In compositions for topical administration, the mixtures are preferably formulated as aqueous solutions at a pH of 3.5 to 6.5. In an embodiment, the pH is adjusted to between 4 and 5. This pH range may be achieved by the inclusion of suitable acids/bases in the composition.

In methods of treating a mammal for an otic infection using the compounds disclosed herein, a topical composition may comprise one or more of an excipient, an antimicrobial agent, a preservative, a cosolvent, a surfactant, a viscosity agent, and/or a bioadhesive agent, as set forth in detail elsewhere herein. In an embodiment, an otic pharmaceutical preparation is a partially-alcoholic preparation. In an aspect, an otic composition is a zinc acetate composition. In another aspect, an otic composition is an acetic acid composition.

As will be understood by the skilled artisan, inclusion of a percentage of alcohol in the preparation will aid in the solubility of the components, including the steroid and the PVP-I. The alcohol component will also serve as a dehydrating component for the surface to which the preparation is applied. Alcohols useful in the invention include methanol, ethanol, and isopropanol, among others.

In other embodiments, compositions and methods disclosed herein are useful for treatment of other parts of the body, including the nasal passages, sinuses, the skin and the vagina. In another embodiment, compositions and methods disclosed herein are useful for treatment of external parts of the body. In an embodiment, PVP-I is present in a composition for treatment of other parts of the body in the range of about 0.1 %-12.5% or about 1% to about 10%. Other suitable PVP-I concentrations are set forth elsewhere herein. In an embodiment, compositions for treatment of other parts of the body may additionally comprise a steroid, such as, but not limited to, dexamethasone.

In an embodiment, compositions and methods disclosed herein are useful for treating a human. In an embodiment, the human is an adult. In another embodiment, the human is a child. In an embodiment, compositions and methods disclosed herein are pediatric compositions, and methods of pediatric treatment. In an aspect, a pediatric composition contains components, including PVP-I, at concentrations suitable for treating a child. By way of a non-limiting example, a pediatric composition may comprise a lower concentration of PVP-I, steroid, or NSAID, than a comparable composition for use in an adult. In an embodiment, an NSAID is used in place of a steroid in a pediatric composition. In an embodiment, a method is provided for pediatric treatment, comprising treatment of a patient using a composition comprising components, including PVP-I, at concentrations suitable for treating a child. In an embodiment, such concentrations are lower than the concentrations of the same components that would be used to treat an adult. In another embodiment, a method is provided for pediatric treatment, comprising treatment of a patient using a composition comprising PVP-I and an NSAID.

In an embodiment, disclosed herein is a method of preparing a composition comprising PVP-I at a starting concentration between about 0.4% and about 12.5% by weight, wherein after a period of one month after preparing the composition, the PVP-I concentration is at least 98% of the PVP-I starting concentration, and after a period of six months after preparing the composition, the PVP-I concentration is at least 96% of the PVP-I starting concentration. In another embodiment, disclosed herein is a method of preparing a composition comprising PVP-I at a starting concentration between about 0.001% and about 0.6% by weight, wherein after a period of one month after preparing the composition, the PVP-I concentration is at least 93% of the PVP-I starting concentration, and after a period of six months after preparing the composition, the PVP-I concentration is at least 93% of the PVP-I starting concentration.

In an embodiment, the method of preparing a PVP-I composition according to the disclosure herein further comprises the addition of one or more steroids including, but not limited to, dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and any combinations thereof. The steroid may be used any form, and in various modified forms such as acetate forms, and sodium phosphate forms, sodium salts, and the like. In an embodiment, a pharmaceutically acceptable salt of the steroid can be used in the method of preparation.

In an embodiment, the method of preparing a PVP-I composition according to the disclosure herein further comprises the addition of one or more NSAIDS including, but not limited to, amfenac, bromfenac, ketotifen fumarate, diclofenac, diclofenac sodium, flurbiprofen sodium, ketorlac, ketorlac tromethamine, suprofen, celecoxib, naproxen, rofecoxib, or a derivative or combination thereof. Pharmaceutically-acceptable salts of NSAIDS are also contemplated herein.

In another embodiment, a method of making a PVP-I composition further comprises adding one or more components required to prepare a suitable pharmaceutical composition. Such components, described elsewhere herein, include, but are not limited to bioadhesive agents and excipients, as well as components required to prepare the composition as a solution, suspension, emulsion (dispersion), gel, cream, or ointment, or other form for administration.

In an embodiment, a method of making a PVP-I composition comprises storing the prepared composition for a period of time before use to allow stabilization and/or degradation to occur. In an embodiment, the prepared composition is stored for about 1 hour, about 2 hours, about 3 hours, about 4 hours, about 5 hours, about 6 hours, about 12 hours, about 18 hours, or about 24 hours before use. In an embodiment, the prepared composition is stored for about 1 day, about 2 days, about 3 days, about 4 days, about 5 days, about 6 days, or about 7 days before use. In an embodiment, the prepared composition is stored for about 1 week, about 2 weeks, about 3 weeks, about 4 weeks, about 5 weeks, about 6 weeks, about 7 weeks, or about 8 weeks before use. In an embodiment, the prepared composition is stored for about 1 month, about 2 months, about 3 months, about 4 months, about 5 months, about 6 months, about 9 months, or about 12 months before use. In an embodiment, the storage is accompanied by agitation and/or stirring of the composition. In another embodiment, the storage is accompanied by application of heat to the composition.

The invention is further described by the following examples. It should be recognized that variations based on the inventive features are within the skill of the ordinary artisan, and that the scope of the invention should not be limited by the examples. To properly determine the scope of the present disclosure, an interested party should consider the claims herein, and any equivalent thereof. All patents, patent applications, and references cited herein are hereby incorporated by reference in their entirety.

### EXAMPLES

### Example 1: Stability of 0.4% PVP-I Composition

Several preparations of 0.4% PVP-I were stored at 25° C for one month, three months, and six months. After one month, the 0.4% PVP-I preparation retained 93.75% of the starting PVP-I concentration. After three months, the 0.4% PVP-I preparation retained 93.27% of the starting PVP-I concentration. After six months, the 0.4% PVP-I preparation retained 93.22% of the starting PVP-I concentration.

### Example 2: Stability of 1.0% PVP-I Composition

Several preparations of 1.0% PVP-I were stored at 25° C for one month, three months, and six months. After one month, the 1.0% PVP-I preparation retained 98.0% of the starting PVP-I concentration. After three months, the 1.0% PVP-I preparation retained 97.0% of the starting PVP-I concentration. After six months, the 1.0% PVP-I preparation retained 96.0% of the starting PVP-I concentration.

### Example 3: Stability of PVP-I in the Presence of Dexamethasone

Several preparations of PVP-I were stored at various temperatures for periods of one month, three months, six months and twelve months. The results are shown in Table 1. The starting concentration of PVP-I, at the time of preparation of the composition, is referenced as 100 percent. The concentrations at each time point are given as percent of the initial concentration. Formulation A: 0.1% dexamethasone + 0.4% PVP-I; Formulation B: 0.1% dexamethasone + 0.48% PVP-I; Formulation C: 0.1% dexamethasone + 0.6% PVP-I; Formulation D: 0.1% dexamethasone + 1.0% PVP-I.

**Table 1: Stability of PVP-I in the Presence of Dexamethasone**

| Formulation | Storage Temperature | Initial Concentration | 1 Month | 3 Months | 3 Months (2) | 6 Months | 12 Months |
|---|---|---|---|---|---|---|---|
| A | 5°C | 100% | | 94.9 | 96.8 | 98.6 | 94.7 |
| A | 25°C | 100% | 93.8 | 92.4 | 91.3 | 87.6 | 83.4 |
| B | 5°C | 100% | | 97.1 | 96.1 | 96.9 | 95.0 |
| B | 25°C | 100% | 95.7 | 90.5 | 90.1 | 88.8 | 84.9 |
| C | 5°C | 100% | | 95.4 | 95.7 | 9732 | 97.3 |
| C | 25°C | 100% | | 93 | 92.4 | 8839 | 88.6 |
| D | 5°C | 100% | | 99.2 | | 99.1 | 100.5 |
| D | 25°C | 100% | 98.5 | 97.1 | | 96.1 | 93.4 |

A non-exhaustive list of aspects of the disclosure is set out in the following numbered clauses:
Clause 1. A composition suitable for topical administration, comprising povidone-iodine (PVP-I) at a starting concentration between about 0.4% and about 12.5% by weight, wherein after a period of one month after preparing the composition, the PVP-I concentration is at least 98% of the PVP-I starting concentration, and after a period of six months after preparing the composition, the PVP-I concentration is at least 96% of the PVP-I starting concentration.
Clause 2. A composition suitable for topical administration, comprising a mixture of
   a. PVP-I at a starting concentration between about 0.4% and about 12.5% by weight; and
   b. at least one non-steroidal anti-inflammatory (NSAID) selected from the group consisting of amfenac, bromfenac, ketotifen fumarate, diclofenac, diclofenac sodium, flurbiprofen sodium, ketorlac, ketorlac tromethamine, suprofen, celecoxib, naproxen, rofecoxib, and combinations and salts thereof,
   wherein after a period of one month after mixing the NSAID and PVP-I to form the composition, the PVP-I concentration is at least 98% of the PVP-I starting concentration, and after a period of six months after mixing the NSAID and PVP-I to form the composition, the PVP-I concentration is at least 96% of the PVP-I starting concentration.
Clause 3. A composition suitable for topical administration, comprising a mixture of
   a. PVP-I at a starting concentration between about 0.4% and about 12.5% by weight; and
   b. at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof,
   wherein after a period of one month after mixing the steroid and PVP-I to form the composition, the PVP-I concentration is at least 98% of the PVP-I starting concentration, and after a period of six months after mixing the steroid and PVP-I to form the composition, the PVP-I concentration is at least 96% of the PVP-I starting concentration.
Clause 4. A composition suitable for topical administration, comprising a mixture of
   a. PVP-I at a starting concentration between about 0.4% and about 12.5% by weight; and
   b. at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof;
   wherein after a period of one month after mixing the steroid and PVP-I to form the composition, the PVP-I concentration is at least 98% of the PVP-I starting concentration, and after a period of six months after mixing the steroid and PVP-I to form the composition, the PVP-I concentration is at least 96% of the PVP-I starting concentration, further wherein, after a period of one month after mixing the steroid and PVP-I to form the composition, the steroid concentration is at least 90% of the steroid starting concentration.
Clause 5. A method of treating a mammal having an otic infection, the method comprising contacting the ear of the mammal with a composition of one of clauses 1-4.
Clause 6. A composition suitable for topical administration, comprising PVP-I at a starting concentration between about 0.001 % and about 0.6% by weight, wherein after a period of one month after preparing the composition, the PVP-I concentration is at least 93% of the PVP-I starting concentration, and after a period of six months after preparing the composition, the PVP-I concentration is at least 93% of the PVP-I starting concentration.
Clause 7. A composition suitable for topical administration, comprising a mixture of
   a. PVP-I at a starting concentration between about 0.001% and about 0.6% by weight; and
   b. at least one NSAID selected from the group consisting of amfenac, bromfenac, ketotifen fumarate, diclofenac, diclofenac sodium, flurbiprofen sodium, ketorlac, ketorlac tromethamine, suprofen, celecoxib, naproxen, rofecoxib, and combinations and salts thereof,
   wherein after a period of one month after mixing the NSAID and PVP-I to form the composition, the PVP-I concentration is at least 93% of the PVP-I starting concentration, and after a period of six months after mixing the NSAID and PVP-I to form the composition, the PVP-I concentration is at least 93% of the PVP-I starting concentration.
Clause 8. A composition suitable for topical administration, comprising a mixture of
   a. PVP-I at a starting concentration between about 0.001 % and about 0.6% by weight; and
   b. at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof;
   wherein after a period of one month after mixing the steroid and PVP-I to form the composition, the PVP-I concentration is at least 93% of the PVP-I starting concentration, and after a period of six months after mixing the steroid and PVP-I to form the composition, the PVP-I concentration is at least 93% of the PVP-I starting concentration.
Clause 9. A composition suitable for topical administration, comprising a mixture of
   a. PVP-I at a starting concentration between about 0.001 % and about 0.6% by weight; and
   b. at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof;
   wherein after a period of one month after mixing the steroid and PVP-I to form the composition, the PVP-I concentration is at least 93% of the PVP-I starting concentration, and after a period of six months after mixing the steroid and PVP-I to form the composition, the PVP-I concentration is at least 93% of the PVP-I starting concentration, further wherein, after a period of one month after mixing the steroid and PVP-I to form the composition, the steroid concentration is at least 90% of the steroid starting concentration.
Clause 10. A method for treating an eye disorder or a microorganism infection of at least one tissue of the eye comprising the step of administering one or more doses of a composition of one of clauses 6-9 to said eye.
Clause 11. An ophthalmic composition suitable for topical administration to an eye, effective for treatment and/or prophylaxis of a microorganism infection or a disorder of at least one tissue of the eye, comprising a mixture of
   a. PVP-I at a starting concentration between about 0.4% and about 1.0% by weight; and
   b. at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof;
   wherein after a period of one month after mixing the steroid and PVP-I to form the composition, the PVP-I concentration is at least 98% of the PVP-I starting concentration, and after a period of six months after mixing the steroid and PVP-I to form the composition, the PVP-I concentration is at least 96% of the PVP-I starting concentration.
Clause 12. An ophthalmic composition suitable for topical administration to an eye, effective for treatment and/or prophylaxis of a microorganism infection or a disorder of at least one tissue of the eye, comprising a mixture of
   a. PVP-I at a starting concentration between about 0.1% and about 0.6% by weight; and
   b. at least one steroid selected from the group consisting of dexamethasone, dexamethasone alcohol, dexamethasone sodium phosphate, fluromethalone acetate, fluromethalone alcohol, lotoprednol etabonate, medrysone, prednisolone acetate, prednisolone sodium phosphate, difluprednate, rimexolone, hydrocortisone, hydrocortisone acetate, lodoxamide tromethamine, and combinations and salts thereof;
   wherein after a period of one month after mixing the steroid and PVP-I to form the composition, the PVP-I concentration is at least 93% of the PVP-I starting concentration, and after a period of six months after mixing the steroid and PVP-I to form the composition, the PVP-I concentration is at least 93% of the PVP-I starting concentration.
Clause 13. A composition suitable for topical administration, comprising PVP-I at a starting concentration between about 0.4% and about 12.5% by weight, wherein after a period of one month after preparation of the composition, the PVP-I concentration is within about 2% to about 3% of the PVP-I starting concentration, and after a period of six months after preparation of the composition, the PVP-I concentration is within about 2% to about 3% of the PVP-I concentration at one month after preparation of the composition.
Clause 14. A composition suitable for topical administration, comprising PVP-I at a starting concentration between about 0.001 % and about 0.6% by weight, wherein after a period of one month after preparation of the composition, the PVP-I concentration is about 5% to about 10% below the PVP-I starting concentration, and after a period of six months after preparation of the composition, the PVP-I concentration is within about 1% of the PVP-I concentration at one month after preparation of the composition.

## Claims

1. A composition suitable for topical administration, comprising a mixture of
a) PVP-I at a starting concentration of between about 0.4% and about 12.5% by weight; and
b) at least one non-steroidal anti-inflammatory (NSAID) selected from the group consisting of amfenac, bromfenac, ketotifen fumarate, diclofenac, diclofenac sodium, flurbiprofen sodium, ketorlac, ketorlac tromethamine, suprofen, celecoxib, naproxen, rofecoxib, and combinations and salts thereof,
wherein after a period of one month after mixing the NSAID and PVP-I to form the composition, the PVP-I concentration is at least 98% of the PVP-I starting concentration, and after a period of six months after mixing the NSAID and PVP-I to form the composition, the PVP-I concentration is at least 96% of the PVP-I starting concentration.

2. The composition of claim 1 for use in a method of treating an eye disorder or a microorganism infection of at least one tissue of the eye wherein said method comprises the step of administering one or more doses of said composition to said eye.

3. The composition of claim 1 or the composition for use as claimed in claim 2, wherein the composition is an ophthalmic composition.

4. The composition of claim 1 or the composition for use as claimed in claim 2, wherein the NSAID is bromfenac.

5. The composition of claim 1 or the composition for use as claimed in claim 2, wherein the NSAID is present at a concentration of about 0.001% to about 10%.

6. The composition of claim 1 or the composition for use as claimed in claim 2, wherein the NSAID is present at a concentration of about 0.1%

7. The composition of claim 1 or the composition for use as claimed in claim 2, wherein the PVP-I concentration is about 12.5%.
